# FASCICULE DE BREVET EUROPEEN

(11) **EP 4 244 870 B1**
(45) Date de publication et mention de la délivrance du brevet: **09.12.2026**
(21) Numéro de dépôt: 21819933.9
(22) Date de dépôt: 16.11.2021
(51) Int. Cl.: G16H 50/80, G16B 20/40

(54) **PROCÉDÉ DE DÉTECTION D'UNE TRANSMISSION INFECTIEUSE DANS UNE POPULATION**
VERFAHREN ZUM NACHWEIS EINER INFEKTIÖSEN ÜBERTRAGUNG IN EINER POPULATION
METHOD FOR DETECTING AN INFECTIOUS TRANSMISSION IN A POPULATION

(30) Priorité: 16.11.2020 FR 2011693
(43) Date de publication de la demande: 20.09.2023
(73) Titulaire: Hospices Civils de Lyon, 69002 Lyon (FR); Université Claude Bernard Lyon 1, 69100 Villeurbanne (FR); Ecole Normale Supérieure de Lyon, 69007 Lyon (FR); Centre National de la Recherche Scientifique (CNRS), 75016 Paris (FR); Institut National de la Santé et de la Recherche Médicale (INSERM), 75013 Paris (FR)
(72) Inventeur: RASIGADE, Jean-Philippe, 69006 LYON (FR)
(74) Mandataire: Regimbeau
(86) Numéro de dépôt international: PCT/FR2021/052021
(87) Numéro de publication internationale: WO 2022/101596

(56) Documents cités:
- MANIGART OLIVIER ET AL: "A gp41-Based Heteroduplex Mobility Assay Provides Rapid and Accurate Assessment of Intrasubtype Epidemiological Linkage in HIV Type 1 Heterosexual Transmission Pairs", AIDS RESEARCH AND HUMAN RETROVIRUSES., vol. 28, no. 12, 1 December 2012 (2012-12-01), US, pages 1745 - 1755, XP055826876, ISSN: 0889-2229, DOI: 10.1089/aid.2012.0023
- YAN JENNIFER ET AL: "Investigating transmission of Mycobacterium abscessus amongst children in an Australian cystic fibrosis centre", JOURNAL OF CYSTIC FIBROSIS, ELSEVIER, NL, vol. 19, no. 2, 8 March 2019 (2019-03-08), pages 219 - 224, XP086176951, ISSN: 1569-1993, [retrieved on 20190308], DOI: 10.1016/J.JCF.2019.02.011
- SONG JENNY Z. ET AL: "Significance of plasma and peripheral blood mononuclear cell derived HIV-1 sequences in establishing epidemiologic linkage between two individuals multiply exposed to HIV-1", MICROBIAL PATHOGENESIS, vol. 26, no. 6, 1 June 1999 (1999-06-01), US, pages 287 - 298, XP055826922, ISSN: 0882-4010, DOI: 10.1006/mpat.1999.0275
- WHONET-SATSCANVOIR TSUTSUI, A.,YAHARA, K.,CLARK, A.FUJIMOTO, K.KAWAKAMI, S.CHIKUMI, H.STELLING, J.: "Automated détection of outbreaks of anti-microbial-resistant bacteria in Japan", THE JOURNAL OF HOSPITAL INFECTION, vol. 102, no. 2, 2019, pages 226 - 233, XP002803776

## Description

### DOMAINE TECHNIQUE GENERAL

La présente invention concerne le domaine de la surveillance épidémiologique, et concerne en particulier un procédé de détection d'une transmission infectieuse dans une population.

### ETAT DE L'ART

Les bases de la surveillance épidémiologique (détection de nouvelles épidémies et contrôle de leur propagation) peuvent être réduites à la question suivante : si deux isolats de pathogènes sont trouvés chez des individus distincts, quelle est la probabilité que les isolats de pathogènes aient été transmis entre ces individus, que ce soit directement, indirectement ou à partir d'une source commune ?

La notion de transmission est liée à l'existence d'un « ancêtre commun » aux isolats de pathogènes trouvés chez ces individus distincts (dits hôtes), et plus précisément au temps écoulé depuis que les isolats pathogènes ont divergé de leur ancêtre commun, appelé techniquement le temps à l'ancêtre commun le plus récent (TMRCA, Time to the Most Recent Common Ancestor).

Chez les agents pathogènes, chaque événement de divergence détectable coincide avec un événement de transmission dans le cas (le plus fréquent) où le taux de transmission est supérieur au taux d'évolution. C'est pourquoi le TMRCA d'une paire d'isolats d'agents pathogènes donne une estimation de l'âge du dernier événement de transmission dans l'ascendance des isolats d'agents pathogènes. En d'autres termes, le TMRCA peut être interprété comme une estimation de la durée de la chaîne de transmission (directe ou indirecte) impliquant les hôtes des isolats de pathogènes existants.

Un TMRCA court suggère une chaîne de transmission courte et vice-versa. En résumé, la probabilité qu'une paire d'isolats ait été impliquée dans une transmission récente est inversement proportionnelle à leur TMRCA.

On peut par exemple établir qu'une transmission a eu lieu entre les hôtes si le TMRCA est inférieur à un seuil caractéristique du pathogène, par exemple 14 jours pour le coronavirus SARS-CoV-2.

Le TMRCA n'est malheureusement pas observable directement et doit être estimé à partir des caractéristiques observables des isolats d'agents pathogènes, généralement en mesurant la « dissimilarité » entre les isolats.

En particulier, on peut séquencer le génome des isolats, et mesurer la « distance génétique » entre les isolats, c'est-à-dire le nombre de différences entre les séquences d'ADN des isolats. Ces différences s'accumulent en effet à la suite de mutations d'ADN dont le nombre est directement lié au TMRCA.

Cette méthode génétique apporte satisfaction, mais est lente et chère. Il faut généralement plusieurs jours pour avoir les résultats du séquençage, ce qui est problématique dans un contexte épidémique. De plus, les limites de la méthode génétique ne la rendent pas applicable à une surveillance épidémique automatisée sur un grand nombre d'isolats.

Alternativement, on peut utiliser d'autres caractéristiques des isolats pour estimer la probabilité de transmission entre une paire d'isolats. Dans ce cas, les informations génotypiques non disponibles sont remplacées par des informations phénotypiques disponibles générées dans le cadre des diagnostics microbiologiques, facilement obtenables. Les informations phénotypiques incluent généralement les espèces des isolats et leur profil de sensibilité aux antimicrobiens. Le problème est qu'on peut difficilement calculer une dissimilarité, et on se contente de considérer que seuls des isolats qui partagent des profils identiques ont été transmis récemment. Cette approche « identité implique transmission » est la pierre angulaire des méthodes actuelles de détection automatisée des épidémies à l'aide de données phénotypiques (Approche WHONET-SaTSCan, voir Tsutsui, A., Yahara, K., Clark, A., Fujimoto, K., Kawakami, S., Chikumi, H., ... Stelling, J. (2019). Automated detection of outbreaks of antimicrobial-resistant bacteria in Japan. The Journal of Hospital Infection, 102(2), 226-233. doi: 10.1016/j.jhin.2018.10.005*).*

Cette approche est bien plus simple mais moins fiable. Contrairement à la méthode génétique qui est de nature quantitative (car un certain nombre de différences génétiques doivent être atteintes pour exclure la transmission (David et al., 2019)), l'approche « identité implique transmission » est binaire et peut exclure à tort des transmissions sur la base de marqueurs qui évoluent très rapidement tels que les résistances aux antimicrobiens. La résistance à plusieurs antimicrobiens peut pourtant émerger et régresser chez un même patient (Choi et al., 2008) et des isolats liés épidémiologiquement peuvent présenter différents profils de résistance. Inversement, certains agents pathogènes évoluent très lentement et même l'identité n'est pas suffisante pour garantir l'existence d'une transmission.

Il serait par conséquent souhaitable de disposer d'une nouvelle solution de détection d'une transmission infectieuse dans une population, qui soit aussi fiable que les méthodes génétiques, tout en étant plus simple, plus efficace et moins coûteuse.

### PRESENTATION DE L'INVENTION

Selon un premier aspect, la présente invention concerne un procédé de détection d'une transmission infectieuse dans une population, le procédé étant caractérisé en ce qu'il comprend la mise en œuvre, par des moyens de traitement de données d'un client, d'étapes de :
(b) Pour une pluralité d'isolats d'agent infectieux chacun associé à un individu de ladite population, obtention d'un vecteur de valeurs descriptives dudit isolat ;
(c) Pour chaque paire d'un premier isolat et d'un deuxième isolat de ladite pluralité respectivement associés à un premier individu et un deuxième individu de ladite population :
   (c1) calcul d'un premier nombre d'isolats correspondant au nombre d'isolats de ladite pluralité présentant une distance avec le premier isolat inférieure ou égale à une distance de référence entre les premier et deuxième isolats, et d'un deuxième nombre d'isolats correspondant au nombre d'isolats de ladite pluralité présentant une distance avec le deuxième isolat inférieure ou égale à ladite distance de référence entre les premier et deuxième isolats, chaque distance entre deux isolats étant représentative d'une dissimilarité entre les vecteurs de valeurs descriptives de ces deux isolats ;
   (c2) estimation d'une probabilité de transmission infectieuse directe entre les premier et deuxième individus en fonction desdits premiers et deuxième nombres d'isolats ;
(d) Détection ou non d'une transmission infectieuse dans ladite population en fonction des probabilités estimées de transmission infectieuse directe entre chaque paire d'individus.

Selon des caractéristiques avantageuses et non-limitatives :
L'étape (c) comprend une sous-étape préalable (c0) de calcul de la distance de référence entre les premier et deuxième isolats.

Ladite probabilité de transmission infectieuse directe entre les premier et deuxième individus décroit lorsque le premier et/ou le deuxième nombre d'isolats croit.

Ladite probabilité T_{XY} de transmission infectieuse directe entre les premier et deuxième individus est donnée par la formule T_{XY}=1/(1+n(X)+n(Y)), où n(X) est le premier nombre d'isolats et n(Y) est le deuxième nombre d'isolats.

Ledit vecteur de valeurs descriptives d'un isolat comprend un profil de résistance de l'isolat et/ou une liste de pics protéiques de l'isolat.

L'étape (b) comprend, pour chaque isolat de ladite pluralité, la mise en œuvre d'un antibiogramme pour déterminer ledit profil de résistance de l'isolat et/ou la mise en œuvre d'une analyse par spectrométrie de masse de l'isolat pour déterminer ladite liste de pics protéiques de l'isolat.

Le procédé comprend une étape (a) d'obtention de ladite pluralité d'isolats à partir de prélèvements biologiques sur une pluralité d'individus de ladite population.

Une transmission infectieuse dans ladite population est détecté à l'étape (d) s'il existe au moins une paire d'un premier isolat et d'un deuxième isolat de ladite pluralité respectivement associés à un premier individu et un deuxième individu de ladite population telle que ladite probabilité de transmission infectieuse directe entre les premier et deuxième individus estimée à l'étape (c2) est supérieure à un seuil de décision.

L'étape (c) est également mise en œuvre pour chaque paire d'un premier isolat et d'un deuxième isolat de ladite pluralité associés à un même individu de ladite population de sorte à estimer une probabilité de référence, l'étape (d) comprenant le calcul dudit seuil de décision en fonction desdites probabilités de transmission infectieuse directe et desdites probabilités de référence.

L'étape (d) comprend la mise en œuvre d'une action sur ladite population en fonction du résultat de la détection.

Selon un deuxième aspect, l'invention concerne un équipement de détection d'une transmission infectieuse dans une population, caractérisé en ce qu'il comprend des moyens de traitement de données configurés pour implémenter des étapes de :
(b) Pour une pluralité d'isolats d'agents infectieux chacun associé à un individu de ladite population, obtention d'un vecteur de valeurs descriptives dudit isolat ;
(c) Pour chaque paire d'un premier isolat et d'un deuxième isolat de ladite pluralité respectivement associés à un premier individu et un deuxième individu de ladite population :
   (c1) calcul d'un premier nombre d'isolats correspondant au nombre d'isolats de ladite pluralité présentant une distance avec le premier isolat inférieure ou égale à une distance de référence entre les premier et deuxième isolats, et d'un deuxième nombre d'isolats correspondant au nombre d'isolats de ladite pluralité présentant une distance avec le deuxième isolat inférieure ou égale à ladite distance de référence entre les premier et deuxième isolats, chaque distance entre deux isolats étant représentative d'une dissimilarité entre les vecteurs de valeurs descriptives de ces deux isolats ;
   (c2) estimation d'une probabilité de transmission infectieuse directe entre les premier et deuxième individus en fonction desdits premiers et deuxième nombres d'isolats ;
(d) Détection ou non d'une transmission infectieuse dans ladite population en fonction des probabilités estimées de transmission infectieuse directe entre chaque paire d'individus.

Selon un troisième aspect, l'invention concerne un système comprenant un équipement selon le deuxième aspect et un serveur comprenant des moyens de stockage de données stockant une base de données d'isolats d'agents infectieux, et optionnellement un équipement d'analyse biologique d'isolats d'agents infectieux.

Selon un quatrième et un cinquième aspect, l'invention concerne un produit programme d'ordinateur comprenant des instructions de code pour l'exécution d'un procédé selon le premier aspect de détection d'une transmission infectieuse dans une population ; et un moyen de stockage lisible par un équipement informatique sur lequel un produit programme d'ordinateur comprend des instructions de code pour l'exécution d'un procédé selon le deuxième aspect de détection d'une transmission infectieuse dans une population.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- la figure 1 est un schéma d'une architecture pour la mise en œuvre du procédé selon l'invention ;
- la figure 2 illustre schématiquement les étapes d'un mode de réalisation préféré du procédé selon l'invention ;
- la figure 3 représente trois exemples de distribution d'isolat et les valeurs de premier et deuxième nombre d'isolats et la probabilité de transmission estimée selon un mode de réalisation du procédé selon l'invention.

### DESCRIPTION DETAILLEE

### Architecture

L'invention concerne un procédé de détection d'une transmission infectieuse dans une population. « Infectieux » fait ici référence à toute infection transmissible, ladite infection impliquant un agent infectieux, i.e. un pathogène, en particulier un virus, une bactérie, un parasite, un champignon, etc. Dans la suite de la description, on fera référence à des isolats d'agents infectieux, c'est-à-dire des échantillons isolés chacun d'un agent infectieux à partir d'un prélèvement biologique sur un individu de ladite population. Ainsi, chaque isolat est associé à un individu de ladite population, dit « hôte » de l'isolat. On note qu'il est tout à fait possible d'avoir plusieurs isolats associés à un même individu, en particulier au cours du temps (cas de prélèvements multiples à des dates différentes), et c'est même souhaitable pour la mise en œuvre d'un mode de réalisation préféré de l'invention, voir plus loin. On note également que l'isolat pathogène peut exister à l'état commensal chez l'hôte, qui ne présente pas nécessairement de maladie clinique.

Par transmission infectieuse dans la population, on entend l'existence d'au moins un couple P_{X}, P_{Y} d'individus dans la population P (P_{X}, P_{X} ∈ P) tel que P_{X} et P_{Y} partagent une même origine infectieuse, i.e. soit P_{X} a transmis directement l'infection à P_{Y}, soit P_{Y} a transmis directement l'infection à P_{X}, soit P_{X} et P_{Y} ont été infectés de manière concomitante (par exemple en ayant touché un même objet), en d'autres termes l'existence d'une contamination impliquant à la fois P_{X} et P_{Y}. On parle de transmission infectieuse directement entre P_{X} et P_{Y}. On peut également dire que P_{X} et P_{Y} font partie d'un même « cluster ». Cela s'oppose à un cas dans lequel P_{X} et P_{Y} aurait contracté l'infection de manière indépendante via un hôte intermédiaire, par exemple si deux individus P_{X'} et P_{Y'} - potentiellement ne faisant pas partie de ladite population - avaient été infectés de manière concomitante mais P_{X'} avait infecté P_{X} et P_{Y'} avait infecté P_{Y}. Dans la suite de la présente description, on appellera par commodité P_{X} le « premier individu » et P_{Y} le « second individu ».

Comme expliqué dans l'introduction, l'existence ou non d'une transmission directe entre P_{X} et P_{Y} est liée à la longueur de la chaîne de transmission, et ainsi au caractère « récent » de l'événement d'origine ayant mené à l'infection conjointe de P_{X} et P_{Y}, c'est-à-dire dans une fenêtre temporelle donnée. Par exemple, dans le cas d'une transmission via P_{X'} et P_{Y'}, la chaîne de transmission est plus longue, et l'événement d'origine (contamination de P_{X'} et P_{Y'}) est « ancien » de sorte qu'il n'existe pas de transmission directe entre P_{X} et P_{Y} au sens de l'invention.

Pour reformuler, on peut considérer que la propriété « il existe une transmission directe entre P_{X} et P_{Y} » est équivalente à « le TMRCA est inférieur à un seuil donné ».

Le résultat de ladite détection peut être binaire (transmission avérée ou non), ou probabiliste, i.e. le présent procédé estime une probabilité de transmission infectieuse directe entre individus dans ladite population.

Ladite population est un ensemble d'individu identifiés, par exemple les patients d'une unité de soin, d'un service, d'un hôpital, d'un établissement médico-social, etc. Comme l'on verra, le présent procédé est particulièrement performant et peut s'appliquer à des populations élevées et impliquer plusieurs milliers voire dizaines de milliers d'isolats (typiquement 100000 isolats par an sur un hôpital - alors que les méthodes génétiques plafonnaient à 300 isolats).

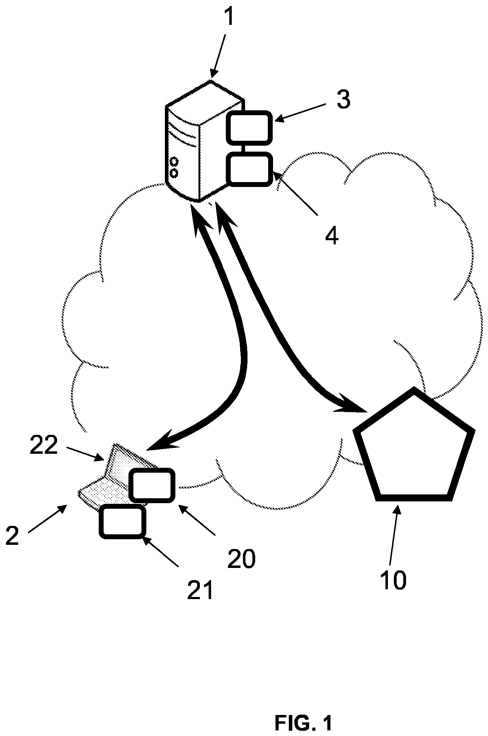

Le présent procédé est mis en œuvre au sein d'une architecture telle que représentée par la **Figure 1****,** grâce à un serveur 1 et un client 2. Le serveur 1 est un équipement de gestion de données de patient, par exemple un serveur central d'un hôpital, et le client 2 est un équipement d'utilisation (mettant en œuvre le procédé de détection), par exemple un autre serveur d'un médecin ou d'un hôpital.

Il est tout à fait possible que les deux équipements 1, 2 soient confondus sous la forme d'un équipement distant, généralement sécurisé pour des questions de confidentialité des données, avec un accès depuis un terminal tel qu'un équipement grand public, notamment un ordinateur de bureau, un portable, etc. Le serveur 1 est typiquement connecté à des équipements d'analyse biologique 10 tel qu'un spectromètre de masse, voir plus loin.

Dans tous les cas, chaque équipement 1, 2 est typiquement un équipement informatique distant relié à un réseau local ou un réseau étendu tel que le réseau internet pour l'échange des données. Chacun comprend des moyens de traitement de données 3, 20 de type processeur, et des moyens de stockage de données 4, 21 telle qu'une mémoire informatique, par exemple une mémoire flash ou un disque dur. Le client 2 comprend typiquement une interface utilisateur 22 telle qu'un écran pour interagir, même si comme expliqué elle peut être déportée sur un terminal distant.

Le serveur 1 stocke avantageusement comme l'on verra une base de données d'isolats d'agents infectieux, dans laquelle chaque isolat est représenté par un vecteur de valeurs descriptives dudit isolat. On répète que par isolat on entend comme son nom l'indique un échantillon isolé d'un agent infectieux dans un prélèvement biologique (i.e. une population homogène de cet agent infectieux - par exemple une cellule bactérienne unique ou une colonie bactérienne), et ledit vecteur d'un isolat décrit en particulier l'agent infectieux de cet isolat.

De manière connue, ledit vecteur de valeurs descriptives d'un isolat peut comprendre un profil de résistance de l'isolat (catégoriel (sensible/intermédiaire/résistant) et/ou quantitatif (concentration minimale inhibitrice)) et/ou un spectre tel qu'une liste de pics protéiques de l'isolat (par exemple issu de l'analyse par spectrométrie de masse, notamment MALDI-ToF).

A noter que ledit vecteur de valeurs descriptives d'un isolat peut alternativement ou en complément comprendre des caractéristiques morphologiques des colonies de l'isolat (pigmentation, aspect muqueux), voire des informations génotypiques de l'isolat (séquences d'ADN).

Le présence de séquences d'ADN peut paraître surprenante dans le contexte où l'on cherche justement à s'éviter le recours à du séquençage, mais si des informations génotypiques sont disponibles on peut parfaitement les utiliser (par exemple, des méthodes en cours de développement vont être amenées à générer des séquences d'ADN des isolats dans un but diagnostic), et on répète qu'elles ne sont absolument pas obligatoires.

### Isolats

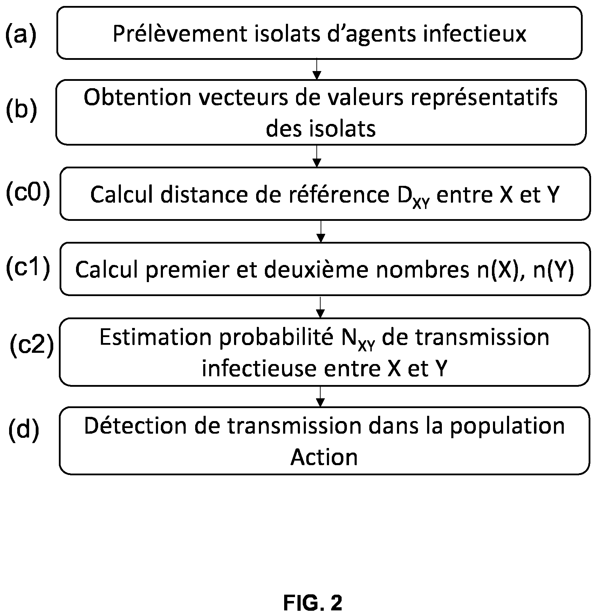

On suppose qu'on dispose d'une pluralité d'isolats. En référence à la **Figure 2****,** le procédé peut commencer directement par une étape (b) d'obtention, par les moyens de traitement de données 20 du client 2, pour une pluralité d'isolats d'agent infectieux chacun associé à un individu de ladite population, d'un vecteur de valeurs descriptives dudit isolat.

Ces vecteurs de valeurs peuvent être déjà stockés sur les moyens de stockage de données 4 du serveur 1 (en particulier dans la base de données évoquée), auquel cas cette étape n'est qu'un chargement de ces vecteurs, mais alternativement (en particulier en cas d'isolats frais, par exemple du jour) comprend pour au moins un isolat la détermination du vecteur de valeurs descriptives de l'isolat, en particulier au moyen de l'équipement d'analyse 10 et/ou des moyens de traitement de données 3 du serveur 1.

Plus précisément, on vient analyser biologiquement l'isolat pour déterminer son vecteur de valeurs. Dans le cas où ledit vecteur de valeurs descriptives d'un isolat comprend un profil de résistance de l'isolat et/ou une liste de pics protéiques de l'isolat, l'étape (a) peut comprendre la mise en œuvre d'un antibiogramme pour déterminer ledit profil de résistance de l'isolat et/ou la mise en œuvre d'une analyse par spectrométrie de masse de l'isolat pour déterminer ladite liste de pics protéiques de l'isolat, comme expliqué typiquement grâce à l'équipement d'analyse biologique 10, mais également potentiellement la mise en œuvre d'une analyse génétique et/ou une observation de l'isolat (par exemple au microscope).

Les résultats peuvent être récupérés par le serveur 1 (avantageusement automatiquement) et stockés dans lesdits moyens de stockage de données 4 du serveur 1 de sorte à enrichir ladite base de données.

Naturellement, l'étape (b) peut être précédée d'une étape (a) d'obtention de ladite pluralité d'isolats à partir de prélèvements biologiques sur une pluralité d'individus de ladite population. En pratique il est nécessaire que chaque isolat soit physiquement obtenu une fois, et ensuite lorsque le vecteur de valeurs descriptives de l'isolat est généré et mis à disposition (en particulier sur le serveur 2), il peut être réutilisé à l'infini.

La présente invention s'inscrit typiquement dans le contexte d'un hôpital dans lequel de nombreux prélèvement sont faits tous les jours sur un certain nombre de patients, permettant de constituer autant d'isolats.

On suppose donc à l'issue de l'étape (b) que lesdits moyens de traitement de données 20 du client 2 ont accès auxdits vecteurs.

### Métrique de densité de voisinage

Le présent procédé propose une métrique innovante, dit N-métrique (pour « neighborhood metric ») ou métrique de densité de voisinage, permettant d'estimer efficacement la probabilité d'une transmission infectieuse directe entre deux individus de ladite population, sans la nécessité d'analyses génétiques. Les analyses évoquées avant (antibiogramme et/ou spectrométrie de masse) sont en effet bien plus facilement accessibles, rapides et peu couteuses. Ladite probabilité de transmission directe entre deux individus est, techniquement, la probabilité qu'il n'existe pas d'hôte intermédiaire entre lesdits individus dans la population. Cela revient à faire l'hypothèse simplificatrice que toute transmission a lieu dans cette population ou, de façon équivalente, qu'aucun individu de la population ne peut être infecté par un tiers en dehors de la population.

L'idée est de constater que la probabilité que deux isolats « similaires » (en termes phylogénétiques) appartiennent à une chaîne de transmission dépend de la distribution des marqueurs dans la population d'agents infectieux : deux isolats similaires avec un phénotype fréquent sont beaucoup moins susceptibles d'être liés épidémiologiquement que deux isolats similaires avec un phénotype très rare. Par exemple, une paire d'isolats de *Serratia marcescens* phénotypiquement identiques et inhabituels est plus susceptible de partager un lien de transmission qu'une paire identique d'*Escherichia coli* omniprésente, car ces derniers ont beaucoup plus de parents potentiels, conformément au bon sens dans la pratique clinique.

La présente invention utilise astucieusement une approche quantitative et probabiliste pour dériver la probabilité de transmission, en considérant la distribution des phénotypes dans la population d'agents infectieux lors de l'estimation de cette probabilité. L'objectif est de proposer une mesure indirecte de la probabilité de transmission directe de deux isolats d'agent infectieux entre deux individus d'une population, en l'absence de séquences d'ADN et d'estimations des TMRCA entre les isolats. L'autre intérêt de cette approche est qu'on obtient une métrique puis une probabilité qui est directement exploitable, et non un temps comme le TMRCA qui doit être comparé à un temps de référence qui reste difficile à déterminer. Plus précisément, la métrique et la probabilité de transmission sont directement liées, et comme l'on verra la probabilité est typiquement inversement proportionnelle à la métrique.

Ainsi, dans une étape (c) principale pour chaque paire d'un premier isolat X et d'un deuxième isolat Y de ladite pluralité respectivement associés à un premier individu Px et un deuxième individu P_{Y} de ladite population P, on calcule ladite métrique de densité de voisinage notée N_{XY} pour estimer la probabilité T_{XY} de transmission infectieuse directe entre les premier et deuxième individus P_{X}, P_{Y}.

L'étape (c) comprend une sous-étape (c1) de calcul d'un premier nombre d'isolats n(X) correspondant au nombre d'isolats de ladite pluralité présentant une distance avec le premier isolat X inférieure ou égale à une distance de référence entre les premier et deuxième isolats X, Y, et un deuxième nombre d'isolats n(Y) correspondant au nombre d'isolats de ladite pluralité présentant une distance avec le deuxième isolat Y inférieure ou égale à ladite distance de référence entre les premier et deuxième isolats X, Y, chaque distance entre deux isolats étant représentative d'une dissimilarité entre les vecteurs de valeurs descriptives de ces deux isolats.

Ladite distance de référence entre les premier et deuxième isolats X, Y, notée D_{XY}, est avantageusement calculée dans une sous-étape préalable (c0).

Par « dissimilarité » entre deux vecteurs, on entend une valeur basse voire nulle lorsque les vecteurs sont identiques, et élevée lorsque les vecteurs sont différents. On ne sera limité à aucune fonction de distance D particulière et on pourra utiliser notamment toute norme sur l'espace des vecteurs de valeurs descriptives d'isolats, et en particulier définir que la distance entre deux isolats est donnée comme la norme de la différence desdits vecteurs, i.e. D_{XY}=∥V_{X}-V_{Y}∥, avec V_{X} et V_{Y} les vecteurs respectifs du premier et du deuxième isolat X, Y.

On peut ainsi par exemple utiliser :
- La distance de Manhattan, i.e. ∥V_{X}-V_{Y}∥=Σ[V_{Xi}-V_{Yi}|
- La distance euclidienne, i.e. ∥V_{X}-V_{Y}∥= √Σ(V_{Xi}-V_{Yi})²
- La distance de Minkowski, i.e. ∥V_{X}-V_{Y}∥=^{p}√Σ|V_{Xi}-V_{Yi}|^{p}
- La distance de Tchebychev, i.e. ∥V_{X}-V_{Y}∥=sup|V_{Xi}-V_{Yi}|
- Etc.

De manière particulièrement préférée, on peut même utiliser une distance composite fonction des valeurs dudit vecteur, i.e. dépendant des paramètres représentés par ces valeurs, typiquement la distance de Gower ou une variante de celle-ci.

Au niveau des taxons, D peut être la divergence phylogénétique connue entre les taxons estimée à partir du taux d'évolution dérivé des protéines ribosomales (voir Jauffrit, F., Penel, S., Delmotte, S., Rey, C., de Vienne, D. M., Gouy, M., ... Brochier-Armanet, C. (2016). RiboDB Database: A Comprehensive Resource for Prokaryotic Systematics. Molecular Biology and Evolution, 33(8), 2170-2172. doi: 10.1093/molbev/msw088*).* Bien que la transmission épidémiologique entre différents taxons ait une probabilité nulle, on peut conserver la divergence inter-taxons pour l'exhaustivité et les développements futurs du modèle qui pourraient impliquer une probabilité de transfert de gènes de résistance horizontale, qui est proportionnelle à la proximité des taxons.

Au niveau du spectre de masse, D peut être la divergence entre les spectres MALDI-ToF (voir Christner, M., Trusch, M., Rohde, H., Kwiatkowski, M., Schlüter, H., Wolters, M., ... Hentschke, M. (2014). Rapid MALDI-TOF mass spectrometry strain typing during a large outbreak of Shiga-Toxigenic Escherichia coli. PloS One, 9(7), e101924. doi: 10.1371/journal.pone.0101924*).* Plusieurs métriques de divergence sont étudiées pour la congruence avec la divergence basée sur le taxon et la résistance.

Au niveau du profil de résistance, D peut être la distance de Gower généralisée entre les profils de résistance par molécule, en utilisant des données quantitatives, notamment les concentrations minimales inhibitrices des molécules, lorsqu'elles sont disponibles.

Au niveau des caractéristiques morphologiques, D peut être soit une distance euclidienne simple si les caractéristiques sont définies par une valeur numérique (par exemple un taux de pigmentation), soit la distance de Manhattan représentant le nombre de caractéristiques qui ne sont pas identiques.

Au niveau des informations génotypiques, D peut être la « distance génétique » entre les isolats, c'est-à-dire le nombre de différences entre les séquences d'ADN des isolats.

Les distances impliquant des valeurs manquantes peuvent être calculées comme la distance moyenne entre les valeurs non manquantes.

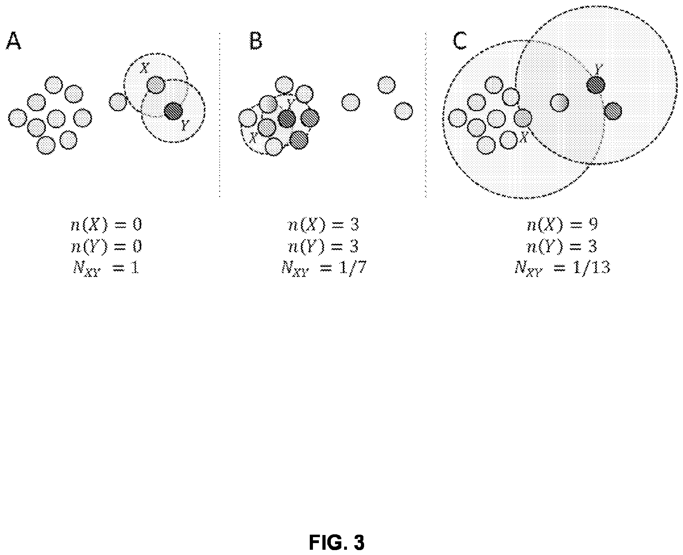

Dans tous les cas, la distance de référence D_{XY} permet de définir un « voisinage » desdits premier et deuxième isolats X, Y, comme l'on voit sur la **Figure 3****.** Dans cette figure, on a représenté pour trois distributions d'isolats un cercle de rayon D_{XY} centré sur chacun des premier et deuxième isolats X, Y. Plus précisément, un isolat dans le cercle de rayon D_{XY} centré sur le premier isolat X présente une distance avec le premier isolat X inférieure ou égale à D_{XY}. Similairement, un isolat dans le cercle de rayon D_{XY} centré sur le deuxième isolat Y présente une distance avec cet isolat inférieure ou égale à D_{XY}. Naturellement, un isolat peut être dans les deux voisinages. A noter que la « distance » est ici toujours en termes de distance vectorielle, i.e. de dissimilarité entre leurs vecteurs.

Lesdits premier et deuxième nombres n(X) et n(Y) correspondent ainsi au nombre d'isolats respectivement dans le voisinage du premier et du deuxième isolat X, Y.

En termes d'interprétation, on comprend que les nombres n(X), n(Y) sont croissants par rapport à D_{XY} (plus les premiers isolats X, Y sont différents, plus leur voisinage est grand, et donc plus la probabilité qu'on rencontre des isolats dans ce voisinage est élevée) et décroissants par rapport à leur rareté (plus un isolat est rare, moins son voisinage contiendra d'autres isolats).

Préférentiellement, on ne compte pas les premiers et deuxièmes isolats X, Y dans leurs voisinages respectifs (mais uniquement les « autres » isolats de ladite pluralité).

Dans la figure 3, l'exemple A montre deux isolats X, Y similaires et rares. On a n(X)=n(Y)=0. L'exemple B montre deux isolats X, Y similaires mais communs. On a n(X)=n(Y)=3. L'exemple C montre deux isolats X, Y dissimilaires, avec X commun. On a n(X)=9 et n(Y)=3.

On comprend qu'il faut réaliser un grand nombre de calculs de distance pour calculer les premier et deuxième nombres. Ainsi on peut optimiser de manière connue en incorporant une matrice de dissimilarité par paire dans un espace euclidien de dimension inférieure par décomposition en vecteurs propres, puis en réduisant les calculs à un problème de recherche classique de voisins proches à rayon fixe résolu en utilisant des méthodes efficaces et parallélisables (par exemple la méthode t-SNE « distributed stochastic neighbor embedding »).

Dans une étape (c2), on estime la probabilité T_{XY} de transmission infectieuse directe entre les premier et deuxième individus en fonction desdits premiers et deuxième nombres d'isolats n(X), n(Y), préférentiellement uniquement en fonction des premier et deuxième nombres n(X), n(Y).

Comme expliqué cette étape (c2) comprend préférentiellement le calcul intermédiaire de la métrique de densité de voisinage N_{XY} associée à la paire d'isolats X, Y, en fonction de leurs premier et deuxième nombres n(X), n(Y), préférentiellement uniquement en fonction des premier et deuxième nombres n(X), n(Y) ; la probabilité T_{XY} de transmission infectieuse directe entre les premier et deuxième individus P_{X}, P_{Y} étant ensuite estimée à partir de la métrique N_{XY}, préférentiellement uniquement en fonction de la métrique N_{XY}. Il reste bien entendu possible de calculer directement la probabilité T_{XY} à partir des premier et deuxième nombres n(X), n(Y).

Le présent procédé n'est pas limité à une formule particulière liant la métrique de densité de voisinage N_{XY} avec n(X) et n(Y), ou à une formule particulière liant la probabilité de transmission T_{XY} avec la métrique N_{XY}, il suffit que T_{XY} décroisse lorsque le premier et/ou le deuxième nombre d'isolats n(X), n(Y) croit, et en particulier que N_{XY} croisse lorsque le premier et/ou le deuxième nombre n(X), n(Y) croit et que T_{XY} décroisse lorsque N_{XY} croît. En effet, le nombre d'isolats dans un voisinage témoigne de la variété d'alternatives possibles à une transmission infectieuse directe entre X et Y. Plus les voisinages sont « peuplés », ou denses, plus l'origine de la transmission dans la population d'hôtes peut être multiple et donc plus une transmission directe entre X et Y est incertaine. Au contraire, si les voisinages sont vides, ou peu denses, cela signifie qu'il n'y a pas d'autres chemins possibles qu'une transmission entre X et Y. En d'autres termes, la vraisemblance d'une transmission directe entre X et Y décroît avec la densité du voisinage de X et Y.

De manière préférée, on prend les formules N_{XY}=1+n(X)+n(Y) et T_{XY}=1/N_{XY}, que l'on combine pour obtenir T_{XY}=1/(1+n(X)+n(Y)). On pourra utiliser de nombreuses autres formules telles que N_{XY}=(1+n(X)(1+n(Y)) et/ou T_{XY}=e^{-N}_{XY}, etc.

Dans la figure 3, l'exemple A montre deux isolats X, Y similaires et rares. On a T_{XY}=1/(1+0+0)=100%. L'exemple B montre deux isolats X, Y similaires mais communs. On a T_{XY}=1/(1+3+3)=14.3%. L'exemple C montre deux isolats X, Y dissimilaires, avec X commun. On a T_{XY}=1/(1+9+2)=8.3%.

Dans une étape (d), on détecte ou non une transmission infectieuse dans ladite population en fonction des probabilités T_{XY} estimées de transmission infectieuse directe entre chaque paire d'individus.

Par exemple, on peut déterminer qu'une transmission a eu lieu s'il existe au moins une paire d'un premier isolat X et d'un deuxième isolat Y pour lesquels la probabilité T_{XY} de transmission infectieuse directe entre les premier et deuxième individus P_{X}, P_{Y} associés à ces isolats est supérieure à un seuil donné, dit seuil de décision, par exemple 80%, même si l'homme du métier pourra utiliser n'importe quel classifieur de son choix.

L'éventuel seuil de décision peut ainsi être prédéterminé, mais alternativement et de manière préférée il peut être également calculé dans l'étape (d), sur la base desdites probabilités T_{XY} mais également de probabilités de transmission infectieuses calculées pour une paire d'isolats de ladite pluralité associés au même individu (i.e. un premier et un deuxième isolat X₁ et X₂ du même individu P_{X}, probabilité notées abusivement T_{XX} pour l'individu P_{X}). On comprendra bien entendu que la notion de « transmission infectieuse directe » ne s'applique plus si le premier et le deuxième individu sont le même, et on parlera plutôt de probabilité « de référence » (pour ledit individu P_{X}).

A ce titre, l'étape (c) est avantageusement également mise en œuvre pour chaque paire d'un premier isolat X₁ et d'un deuxième isolat X₂ de ladite pluralité associés à un même individu de ladite population (i.e. au final pour chaque paire d'isolats possible). On a pour ces paires exactement les mêmes étapes (c1) et (c2).

Ainsi, le seuil de décision peut être obtenu en comparant la distribution des valeurs des probabilités T dans deux sous-ensembles de paires d'isolats issues de ladite pluralité d'isolats, i.e. deux sous-ensembles de l'ensemble des paires possibles, le premier sous-ensemble étant constitué des paires d'isolats issus du même individu (paires dite « intra-patient », probabilités notées T_{XX}) et le second sous-ensemble étant constitué des paires d'isolats issues d'individus différents (paires dites « inter-patients », probabilités notées T_{XY}). Le seuil de décision choisi est par exemple la valeur séparant au mieux les deux groupes de valeurs de probabilités, on pourra l'obtenir par n'importe quelle méthode d'analyse discriminante, par exemple une minimisation de la somme du nombre de T_{XX} inférieures au seuil et de T_{XY} supérieures au seuil.

En effet, naturellement les probabilités T_{XX} de références sont en moyenne plus élevées que les probabilités T_{XY}, puisqu'on peut être quasiment sûr que les deux isolats associés à un même individu partagent une même origine récente. Si la valeur de T_{XY} entre des isolats d'individus différents est comparable aux valeurs de probabilité T_{XX} observées précédemment entre des isolats d'un même individu, alors le TMRCA de cette paire d'isolats d'individus différents est comparable au TMRCA que l'on observerait si les isolats provenaient d'un unique individu : cette situation indique une transmission récente.

Cette méthode préférentielle de détermination du seuil de décision présente l'avantage de pouvoir être appliquée directement à l'ensemble des agent infectieux présents dans la population, à condition qu'un nombre suffisant de paires d'isolats intra-patient et inter-patients soient disponibles, et de façon à ce que le seuil de décision obtenu pour chaque agent infectieux reflète la diversité des profils des isolats (obtenus par les analyses évoquées avant, dont l'antibiogramme et/ou spectrométrie de masse) dans la population au sein de laquelle une transmission infectieuse doit être détectée.

Dans la figure 3, on peut donc conclure à l'existence d'une transmission infectieuse directe entre P_{X} et P_{Y} dans l'exemple A puisque T_{XY}=100%, et donc une transmission dans ladite population est détectée. Cette étape (d) peut comprendre le déclenchement d'une alerte sur l'interface 22 du client 2 mais également la mise en œuvre d'une action sur ladite population en fonction du résultat de la détection, notamment des mesures de lutte contre l'infection en cas de détection avérée, par exemple de l'allocation de ressources à des services d'hygiène et/ou la mise en œuvre de prélèvements à plus grande échelle. En particulier, la détection d'une transmission dans un service d'un hôpital peut entraîner une désinfection complète du service et/ou un test systématique de tous les patients.

Alternativement ou en complément, l'étape (d) peut comprendre la vérification d'une transmission détectée par analyse génétique. En effet il y aura au maximum une poignée de transmissions entre individus avec une probabilité élevée, de sorte que le nombre de tests génétiques reste bas et peut être effectué rapidement.

### Tests

Les performances du présent procédé de détection d'une transmission infectieuse ont été vérifiées sur un modèle de simulation de la transmission d'un agent infectieux dans une population d'hôtes. Ce modèle simule également l'évolution du profil de résistance de l'agent infectieux, représenté sous forme d'un vecteur binaire où la sensibilité à un antibiotique est représenté par 0 et la résistance est représentée par 1. Le profil de résistance binaire est choisi comme la représentation préférée du vecteur de valeurs descriptive de chaque isolat. On utilise typiquement un vecteur de longueur 24, soit 24 antibiotiques testés, en accord avec les pratiques habituelles en bactériologie médicale. De façon importante, le profil de résistance binaire est le type de valeurs descriptives présentant la plus faible résolution (en comparaison de CMI par exemple). Ainsi, ce type de valeurs descriptives est représentatif de conditions d'application défavorables, limitant le risque de surestimation des performances réelles du procédé de détection.

Dans le modèle de simulation, le pathogène évolue sous la forme d'une généalogie (phylogénie) aléatoire. La simulation commence avec une unique lignée (individu) de l'agent infectieux présent chez un unique patient. Au cours du temps, chaque lignée du pathogène donne naissance à deux lignées jusqu'à l'obtention d'un nombre défini d'isolats échantillonnés dans la population. Au cours du temps, chaque lignée du pathogène peut être transmise d'un patient à un autre avec un taux de transmission Rₜᵣₐₙₛ, typiquement Rₜᵣₐₙₛ = 1 (grandeur sans dimension). Chaque lignée de l'agent infectieux peut évoluer vers la résistance (ou perdre cette résistance) à un antibiotique avec un taux d'évolution Rₑᵥₒₗ plus lent que le taux de transmission, typiquement Rₑᵥₒₗ = 0.05. La proportion Pᵣₑₛ de résistance à l'équilibre du modèle représente la pression antibiotique dans la population d'hôte. Cette proportion est typiquement modérée pour des pathogènes hospitaliers, Pᵣₑₛ = 0.25. Ces paramètres permettent d'obtenir des profils de résistance simulés en accord avec les données réelles, dans lesquelles la majorité des agents infectieux sont très sensibles et seuls quelques agents infectieux accumulent de nombreuses résistances.

A l'issue de chaque simulation, la longueur de la chaîne de transmission entre chaque paire d'isolats est calculée. La longueur de la chaîne de transmission est définie comme le nombre de patients différents ayant été colonisés par l'ensemble des ancêtres de chaque isolat jusqu'à leur ancêtre commun le plus récent. Les paires d'isolats présents chez le même patient sont ignorées (chaîne de transmission triviale, de longueur 1). Les paires d'isolats avec une chaîne de transmission de longueur 2 sont catégorisées comme directement transmises. Les paires d'isolats avec une chaîne de transmission de longueur supérieure à 2 sont catégorisées comme non directement transmises, puisqu'un hôte intermédiaire est impliqué dans la transmission. Une fois ces données obtenues, le présent procédé est utiliser pour détecter si une paire d'isolats a été directement transmise, en utilisant comme donnée d'entrée les profils de résistance binaire de la paire d'isolats à l'exclusion de toute autre donnée.

Pour chaque paire d'isolats de patients différents, on prend comme distance de référence D_{XY} la proportion de différences dans le vecteur binaire représentant le profil de résistance (distance de Manhattan).

La métrique de densité de voisinage N_{XY} et la probabilité T_{XY} de transmission infectieuse directe sont calculées comme décrit précédemment (N_{XY}=1+n(X)+n(Y) et T_{XY}=1/N_{XY}).

La simulation est répétée 400 fois sur une population finale de 200 isolats évoluant dans une population de 100 hôtes potentiels. Les mesures de performances (Table 1) montrent que les performances de détection d'une transmission directe par un classificateur basé sur la densité de voisinage N_{XY} sont non seulement élevées (aire sous la courbe ROC > 0,90) mais surpassent les performances d'un classificateur basé sur la dissimilarité (i.e. la distance de référence D_{XY}), avec un odds ratio médian plus élevé d'un facteur 1,64 et des taux inférieurs de faux positifs et de faux négatifs. L'aire sous la courbe ROC du classificateur par densité de voisinage était plus élevée que celle du classificateur par dissimilarité dans 96.1% des simulations.

Ces simulations indiquent que même dans des conditions défavorables, utilisant un vecteur de valeurs descriptives à faible résolution basé sur un antibiogramme, un classificateur basé sur la densité de voisinage permet de détecter les paires d'isolats directement transmises avec des performances élevées, et que ces performances surpassent celles d'un classificateur basé sur une mesure de dissimilarité.

**Table 1**

| | **Prédicteur (médiane et IQR)** | |
|---|---|---|
| | **Dissimilarité** | **N-métrique** |
| Aire sous la courbe | | |
| ROC | 0,88 [0,85 à 0,90] | 0,91 [0,88 à 0,93] |
| Odds ratio | 17,5 [12,8 à 26.0] | 28,7 [19,1 à 43,6] |
| % de faux négatifs | 19,6 [15,2 à 25,5] | 16,5 [11,8 à 21,9] |
| % de faux positifs | 18,4 [14,6 à 22,4] | 14,9 [12,4 à 18,1] |

### Equipement, produit programme d'ordinateur

Selon un deuxième aspect, l'invention concerne un équipement de détection d'une transmission infectieuse dans une population pour la mise en œuvre du procédé selon le premier aspect, i.e. le client 2.

Comme expliqué cet équipement 2 comprend des moyens de traitement de données 20 configurés pour implémenter des étapes de :
(b) Pour une pluralité d'isolats d'agents infectieux chacun associé à un individu de ladite population, obtention d'un vecteur de valeurs descriptives dudit isolat ;
(c) Pour chaque paire d'un premier isolat X et d'un deuxième isolat Y de ladite pluralité respectivement associés à un premier individu Px et un deuxième individu P_{Y} de ladite population :
   (c1) calcul d'un premier nombre d'isolats n(X) correspondant au nombre d'isolats de ladite pluralité présentant une distance avec le premier isolat X inférieure ou égale à une distance de référence D_{XY} entre les premier et deuxième isolats (X, Y (le cas échéant après (c0) de calcul de la distance de référence D_{XY} entre les premier et deuxième isolats X, Y), et d'un deuxième nombre d'isolats n(Y) correspondant au nombre d'isolats de ladite pluralité présentant une distance avec le deuxième isolat Y inférieure ou égale à ladite distance de référence D_{XY} entre les premier et deuxième isolats X, Y, chaque distance entre deux isolats étant représentative d'une dissimilarité entre les vecteurs de valeurs descriptives de ces deux isolats ;
   (c2) estimation d'une probabilité T_{XY} de transmission infectieuse directe entre les premier et deuxième individus en fonction desdits premiers et deuxième nombres d'isolats n(X), n(Y) ;
(d) Détection ou non d'une transmission infectieuse dans ladite population en fonction des probabilités T_{XY} estimées de transmission infectieuse directe entre chaque paire d'individus

Selon un troisième aspect l'invention concerne un système comprenant un équipement 2 selon le deuxième aspect et un serveur 1 comprenant des moyens de stockage de données 4 stockant une base de données d'isolats d'agents infectieux (chacun représenté par un vecteur de valeurs), et optionnellement un équipement d'analyse biologique 10 d'isolats d'agents infectieux, pour la détermination desdits vecteurs de valeurs représentatifs des isolats.

Selon un quatrième et un cinquième aspects, l'invention concerne un produit programme d'ordinateur comprenant des instructions de code pour l'exécution (en particulier sur les moyens de traitement de données 3, 20 du serveur 1 et/ou du client 2) d'un procédé selon le premier aspect de détection d'une transmission infectieuse dans une population, ainsi que des moyens de stockage lisibles par un équipement informatique (une mémoire 4, 21 du serveur 1 et/ou du client 2) sur lequel on trouve ce produit programme d'ordinateur.

## Revendications

1. Procédé de détection d'une transmission infectieuse dans une population, le procédé étant **caractérisé en ce qu'**il comprend la mise en œuvre, par des moyens de traitement de données (20) d'un client (2), d'étapes de :
(b) Pour une pluralité d'isolats d'agent infectieux chacun associé à un individu de ladite population, obtention d'un vecteur de valeurs descriptives dudit isolat, le vecteur de valeurs descriptives d'un isolat comprenant un profil de résistance de l'isolat et/ou une liste de pics protéiques de l'isolat et/ou des caractéristiques morphologiques des colonies de l'isolat et/ou des informations génotypiques de l'isolat ;
(c) Pour chaque paire d'un premier isolat (X) et d'un deuxième isolat (Y) de ladite pluralité respectivement associés à un premier individu et un deuxième individu de ladite population :
(c1) calcul d'un premier nombre d'isolats (n(X)) correspondant au nombre d'isolats de ladite pluralité présentant une distance avec le premier isolat (X) inférieure ou égale à une distance de référence entre les premier et deuxième isolats (X, Y), et d'un deuxième nombre d'isolats (n(Y)) correspondant au nombre d'isolats de ladite pluralité présentant une distance avec le deuxième isolat (Y) inférieure ou égale à ladite distance de référence entre les premier et deuxième isolats (X, Y), chaque distance entre deux isolats étant représentative d'une dissimilarité entre les vecteurs de valeurs descriptives de ces deux isolats ;
(c2) estimation d'une probabilité (T_{XY}) de transmission infectieuse directe entre les premier et deuxième individus en fonction desdits premiers et deuxième nombres d'isolats (n(X), n(Y)), la probabilité (T_{XY}) de transmission infectieuse directe entre les premier et deuxième individus décroit lorsque le premier et/ou le deuxième nombre d'isolats (n(X), n(Y)) croit ;
(d) Détection ou non d'une transmission infectieuse dans ladite population en fonction des probabilités (T_{XY}) estimées de transmission infectieuse directe entre chaque paire d'individus, une transmission infectieuse dans ladite population est détecté à l'étape (d) s'il existe au moins une paire d'un premier isolat (X) et d'un deuxième isolat (Y) de ladite pluralité respectivement associés à un premier individu et un deuxième individu de ladite population telle que la probabilité (T_{XY}) de transmission infectieuse directe entre les premier et deuxième individus estimée à l'étape (c2) est supérieure à un seuil de décision.

2. Procédé selon la revendication 1, dans lequel l'étape (c) comprend une sous-étape préalable (c0) de calcul de la distance de référence (D_{XY}) entre les premier et deuxième isolats (X, Y).

3. Procédé selon l'une des revendications 1 à 2, dans lequel ladite probabilité T_{XY} de transmission infectieuse directe entre les premier et deuxième individus est donnée par la formule T_{XY}=1/(1+n(X)+n(Y)), où n(X) est le premier nombre d'isolats et n(Y) est le deuxième nombre d'isolats.

4. Procédé selon l'une des revendications 1 à 3, dans lequel l'étape (b) comprend, pour chaque isolat de ladite pluralité, la mise en œuvre d'un antibiogramme pour déterminer ledit profil de résistance de l'isolat et/ou la mise en œuvre d'une analyse par spectrométrie de masse de l'isolat pour déterminer ladite liste de pics protéiques de l'isolat et/ou la mise en œuvre d'une analyse génétique de l'isolat pour déterminer lesdites informations génotypiques de l'isolat et/ou une observation de l'isolat pour déterminer lesdites caractéristiques morphologiques des colonies de l'isolat.

5. Procédé selon l'une des revendications précédentes, comprenant une étape (a) d'obtention de ladite pluralité d'isolats à partir de prélèvements biologiques sur une pluralité d'individus de ladite population.

6. Procédé selon l'une des revendications 1 à 5, dans lequel l'étape (c) est également mise en œuvre pour chaque paire d'un premier isolat (X₁) et d'un deuxième isolat (X₂) de ladite pluralité associés à un même individu de ladite population de sorte à estimer une probabilité (T_{XX}) de référence, l'étape (d) comprenant le calcul dudit seuil de décision en fonction desdites probabilités (T_{XY}) de transmission infectieuse directe et desdites probabilités (T_{XX}) de référence.

7. Procédé selon l'une des revendications 1 à 6, dans lequel l'étape (d) comprend la mise en œuvre d'une action sur ladite population en fonction du résultat de la détection.

8. Equipement (2) de détection d'une transmission infectieuse dans une population, **caractérisé en ce qu'**il comprend des moyens de traitement de données (20) configurés pour implémenter des étapes de :
(b) Pour une pluralité d'isolats d'agents infectieux chacun associé à un individu de ladite population, obtention d'un vecteur de valeurs descriptives dudit isolat ;
(c) Pour chaque paire d'un premier isolat (X) et d'un deuxième isolat (Y) de ladite pluralité respectivement associés à un premier individu et un deuxième individu de ladite population :
(c1) calcul d'un premier nombre d'isolats (n(X)) correspondant au nombre d'isolats de ladite pluralité présentant une distance avec le premier isolat (X) inférieure ou égale à une distance de référence entre les premier et deuxième isolats (X, Y), et d'un deuxième nombre d'isolats (n(Y)) correspondant au nombre d'isolats de ladite pluralité présentant une distance avec le deuxième isolat (Y) inférieure ou égale à ladite distance de référence entre les premier et deuxième isolats (X, Y), chaque distance entre deux isolats étant représentative d'une dissimilarité entre les vecteurs de valeurs descriptives de ces deux isolats ;
(c2) estimation d'une probabilité (T_{XY}) de transmission infectieuse directe entre les premier et deuxième individus en fonction desdits premiers et deuxième nombres d'isolats (n(X), n(Y)) ;
(d) Détection ou non d'une transmission infectieuse dans ladite population en fonction des probabilités (T_{XY}) estimées de transmission infectieuse directe entre chaque paire d'individus

9. Système comprenant un équipement (2) selon la revendication 8 et un serveur (1) comprenant des moyens de stockage de données (4) stockant une base de données d'isolats d'agents infectieux, et optionnellement un équipement d'analyse biologique (10) d'isolats d'agents infectieux.

10. Produit programme d'ordinateur comprenant des instructions de code pour l'exécution d'un procédé selon l'une des revendications 1 à 8 de détection d'une transmission infectieuse dans une population, lorsque ledit programme est exécuté sur un ordinateur.

11. Moyen de stockage lisible par un équipement informatique sur lequel un produit programme d'ordinateur comprend des instructions de code pour l'exécution d'un procédé selon l'une des revendications 1 à 8 de détection d'une transmission infectieuse dans une population.

## Patentansprüche

1. Verfahren zum Nachweis einer infektiösen Übertragung in einer Population, wobei das Verfahren **dadurch gekennzeichnet ist, dass** es die Durchführung von folgenden Schritten durch Datenverarbeitungsmittel (20) eines Clients (2) umfasst:
(b) für eine Vielzahl von Isolaten von Infektionserregern, die jeweils einem Individuum der Population zugeordnet sind, Erhalten eines Vektors deskriptiver Werte des Isolats; wobei der Vektor deskriptiver Werte eines Isolats ein Resistenzprofil des Isolats und/oder eine Liste von Proteinpeaks des Isolats und/oder morphologische Merkmale der Kolonien des Isolats und/oder Genotypinformationen des Isolats umfasst;
(c) für jedes Paar aus einem ersten Isolat (X) und einem zweiten Isolat (Y) der Vielzahl, die jeweils einem ersten Individuum und einem zweiten Individuum der Population zugeordnet sind:
(c1) Berechnen einer ersten Anzahl von Isolaten (n(X)), die der Anzahl von Isolaten der Vielzahl entspricht, die einen Abstand zu dem ersten Isolat (X) aufweisen, der kleiner oder gleich einem Referenzabstand zwischen dem ersten und dem zweiten Isolat (X, Y) ist, und einer zweiten Anzahl von Isolaten (n(Y)), die der Anzahl von Isolaten der Vielzahl entspricht, die einen Abstand zu dem zweiten Isolat (Y) aufweisen, der kleiner oder gleich dem Referenzabstand zwischen dem ersten und dem zweiten Isolat (X, Y) ist, wobei jeder Abstand zwischen zwei Isolaten repräsentativ für eine Unähnlichkeit zwischen den Vektoren deskriptiver Werte dieser beiden Isolate ist;
(c2) Schätzen einer Wahrscheinlichkeit (T_{XY}) einer direkten infektiösen Übertragung zwischen dem ersten und dem zweiten Individuum in Abhängigkeit von der ersten und der zweiten Anzahl von Isolaten (n(X), n(Y)); wobei die Wahrscheinlichkeit (T_{XY}) einer direkten infektiösen Übertragung zwischen dem ersten und dem zweiten Individuum abnimmt, wenn die erste und/oder die zweite Anzahl von Isolaten (n(X), n(Y)) zunimmt;
(d) Nachweisen oder Nicht-Nachweisen einer infektiösen Übertragung in der Population in Abhängigkeit von den geschätzten Wahrscheinlichkeiten (T_{XY}) einer direkten infektiösen Übertragung zwischen jedem Paar von Individuen, wobei eine infektiöse Übertragung in der Population in Schritt (d) nachgewiesen wird, wenn mindestens ein Paar aus einem ersten Isolat (X) und einem zweiten Isolat (Y) der Vielzahl existiert, die jeweils einem ersten Individuum und einem zweiten Individuum der Population zugeordnet sind, derart, dass die in Schritt (c2) geschätzte Wahrscheinlichkeit (T_{XY}) einer direkten infektiösen Übertragung zwischen dem ersten und dem zweiten Individuum größer als ein Entscheidungsschwellenwert ist.

2. Verfahren nach Anspruch 1, wobei Schritt (c) einen vorherigen Teilschritt (c0) des Berechnens des Referenzabstands (D_{XY}) zwischen dem ersten und dem zweiten Isolat (X, Y) umfasst.

3. Verfahren nach einem der Ansprüche 1 bis 2, wobei die Wahrscheinlichkeit T_{XY} einer direkten infektiösen Übertragung zwischen dem ersten und dem zweiten Individuum durch die Formel T_{XY}=1/(1+n(X)+n(Y)) gegeben ist, wobei n(X) die erste Anzahl von Isolaten und n(Y) die zweite Anzahl von Isolaten ist.

4. Verfahren nach einem der Ansprüche 1 bis 3, wobei Schritt (b) für jedes Isolat der Vielzahl die Durchführung eines Antibiogramms zur Bestimmung des Resistenzprofils des Isolats und/oder die Durchführung einer massenspektrometrischen Analyse des Isolats zur Bestimmung der Liste von Proteinpeaks des Isolats und/oder die Durchführung einer genetischen Analyse des Isolats zur Bestimmung der genotypischen Informationen des Isolats und/oder eine Betrachtung des Isolats zur Bestimmung der morphologischen Eigenschaften der Kolonien des Isolats umfasst.

5. Verfahren nach einem der vorhergehenden Ansprüche, umfassend einen Schritt (a) des Erhaltens der Vielzahl von Isolaten aus biologischen Proben von einer Vielzahl von Individuen der Population.

6. Verfahren nach einem der Ansprüche 1 bis 5, wobei Schritt (c) ebenfalls für jedes Paar aus einem ersten Isolat (X₁) und einem zweiten Isolat (X₂) der Vielzahl durchgeführt wird, die demselben Individuum der Population zugeordnet sind, um eine Referenzwahrscheinlichkeit (T_{XX}) zu schätzen, wobei Schritt (d) das Berechnen des Entscheidungsschwellenwerts in Abhängigkeit von den Wahrscheinlichkeiten (T_{XY}) einer direkten infektiösen Übertragung und den Referenzwahrscheinlichkeiten (T_{XX}) umfasst.

7. Verfahren nach einem der Ansprüche 1 bis 6, wobei Schritt (d) die Durchführung einer Maßnahme bezüglich der Population in Abhängigkeit von dem Ergebnis des Nachweises umfasst.

8. Ausrüstung (2) zum Nachweis einer infektiösen Übertragung in einer Population, **dadurch gekennzeichnet, dass** sie Datenverarbeitungsmittel (20) umfasst, die konfiguriert sind, um folgende Schritte zu implementieren:
(b) für eine Vielzahl von Isolaten von Infektionserregern, die jeweils einem Individuum der Population zugeordnet sind, Erhalten eines Vektors deskriptiver Werte des Isolats;
(c) für jedes Paar aus einem ersten Isolat (X) und einem zweiten Isolat (Y) der Vielzahl, die jeweils einem ersten Individuum und einem zweiten Individuum der Population zugeordnet sind:
(c1) Berechnen einer ersten Anzahl von Isolaten (n(X)), die der Anzahl von Isolaten der Vielzahl entspricht, die einen Abstand zu dem ersten Isolat (X) aufweisen, der kleiner oder gleich einem Referenzabstand zwischen dem ersten und dem zweiten Isolat (X, Y) ist, und einer zweiten Anzahl von Isolaten (n(Y)), die der Anzahl von Isolaten der Vielzahl entspricht, die einen Abstand zu dem zweiten Isolat (Y) aufweisen, der kleiner oder gleich dem Referenzabstand zwischen dem ersten und dem zweiten Isolat (X, Y) ist, wobei jeder Abstand zwischen zwei Isolaten repräsentativ für eine Unähnlichkeit zwischen den Vektoren deskriptiver Werte dieser beiden Isolate ist;
(c2) Schätzen einer Wahrscheinlichkeit (T_{XY}) einer direkten infektiösen Übertragung zwischen dem ersten und dem zweiten Individuum in Abhängigkeit von der ersten und der zweiten Anzahl von Isolaten (n(X), n(Y));
(d) Nachweisen oder Nicht-Nachweisen einer infektiösen Übertragung in der Population in Abhängigkeit von den geschätzten Wahrscheinlichkeiten (T_{XY}) einer direkten infektiösen Übertragung zwischen jedem Paar von Individuen.

9. System, umfassend eine Ausrüstung (2) nach Anspruch 8 und einen Server (1), der Datenspeichermittel (4) umfasst, die eine Datenbank von Isolaten von Infektionserregern speichern, und optional eine Ausrüstung zur biologischen Analyse (10) von Isolaten von Infektionserregern.

10. Computerprogrammprodukt, umfassend Codeanweisungen zur Ausführung eines Verfahrens nach einem der Ansprüche 1 bis 8 zum Nachweis einer infektiösen Übertragung in einer Population, wenn das Programm auf einem Computer ausgeführt wird.

11. Von einer Computerausrüstung lesbares Speichermedium, auf dem ein Computerprogrammprodukt Codeanweisungen zur Ausführung eines Verfahrens nach einem der Ansprüche 1 bis 8 zum Nachweis einer infektiösen Übertragung in einer Population umfasst.

## Claims

1. A method for detecting an infectious transmission in a population, the method being **characterized in that** it comprises implementing, by data processing means (20) of a client (2), steps of:
(b) For a plurality of infectious agent isolates each associated with an individual of said population, obtaining a vector with values descriptive of said isolate, the vector with values descriptive of an isolate comprises a resistance profile of the isolate and/or a list of protein peaks of the isolate and/or of the morphological characteristics of the colonies of the isolate and/or genotypic information of the isolate;
(c) for each pair of a first isolate (X) and a second isolate (Y) of said plurality respectively associated with a first individual and a second individual of said population:
(c1) calculating a first number of isolates (n(X)) corresponding to the number of isolates of said plurality having a distance to the first isolate (X) less than or equal to a reference distance between the first and second isolates (X, Y), and a second number of isolates (n(Y)) corresponding to the number of isolates of said plurality having a distance to the second isolate (Y) less than or equal to said reference distance between the first and second isolates (X, Y), each distance between two isolates being representative of a dissimilarity between the vectors of values descriptive of these two isolates;
(c2) estimating a probability (T_{XY}) of direct infectious transmission between the first and second individuals as a function of said first and second number of isolates (n(X), n(Y)), the probability (T_{XY}) of direct infectious transmission between the first and second individuals decreases when the first and/or the second number of isolates (n(X), n(Y)) increases;
(d) Detecting or not detecting an infectious transmission in said population as a function of the estimated probabilities (T_{XY}) of direct infectious transmission between each pair of individuals, an infectious transmission in said population is detected in step (d) if there is at least one pair of a first isolate (X) and a second isolate (Y) of said plurality respectively associated with a first individual and a second individual of said population such that said probability (T_{XY}) of direct infectious transmission between the first and second individuals estimated in step (c2) is greater than a decision threshold.

2. The method according to claim 1, wherein step (c) comprises a preliminary sub-step (c0) of calculating the reference distance (D_{XY}) between the first and second isolates (X, Y).

3. The method according to claims 1 to 2, wherein said probability T_{XY} of direct infectious transmission between the first and second individuals is given by the formula T_{XY} =1/(1+n(X)+n(Y)), where n(X) is the first number of isolates and n(Y) is the second number of isolates.

4. The method according to claims 1 to 3, wherein step (b) comprises, for each isolate of said plurality, the implementation of an antibiogram to determine said resistance profile of the isolate and/or the implementation of a mass spectrometry analysis of the isolate to determine said list of protein peaks of the isolate and/or the implementation of a genetic analysis of the isolate to determine said genotypic information of the isolate and/or an observation of the isolate to determine said morphological characteristics of the colonies of the isolate.

5. The method according to one of the preceding claims, comprising a step (a) of obtaining said plurality of isolates from biological specimens taken from a plurality of individuals of said population.

6. The method according to one of claims 1 to 5, wherein step (c) is also implemented for each pair of a first isolate (X₁) and a second isolate (X₂) of said plurality associated with the same individual of said population so as to estimate a reference probability (T_{XX}), step (d) comprising the calculation of said decision threshold as a function of said probabilities (T_{XY}) of direct infectious transmission and of said reference probabilities (T_{XX}).

7. The method according to one of claims 1 to 6, wherein step (d) comprises the implementation of an action on said population according to the result of the detection.

8. An item of equipment (2) for detecting an infectious transmission in a population, **characterized in that** it comprises data processing means (20) configured to implement steps of:
(b) For a plurality of infectious agent isolates each associated with an individual of said population, obtaining a vector with values descriptive of said isolate;
(c) for each pair of a first isolate (X) and a second isolate (Y) of said plurality respectively associated with a first individual and a second individual of said population:
(c1) calculating a first number of isolates (n(X)) corresponding to the number of isolates of said plurality having a distance to the first isolate (X) less than or equal to a reference distance between the first and second isolates (X, Y), and a second number of isolates (n(Y)) corresponding to the number of isolates of said plurality having a distance to the second isolate (Y) less than or equal to said reference distance between the first and second isolates (X, Y), each distance between two isolates being representative of a dissimilarity between the vectors of values descriptive of these two isolates;
(c2) estimating a probability (T_{XY}) of direct infectious transmission between the first and second individuals as a function of said first and second number of isolates (n(X), n(Y));
(d) Detecting or not detecting an infectious transmission in said population as a function of the estimated probabilities (T_{XY}) of direct infectious transmission between each pair of individuals

9. A system comprising an item of equipment (2) according to claim 8 and a server (1) comprising data storage media (4) storing a database of infectious agent isolates, and optionally an item of equipment (10) for the biological analysis of infectious agent isolates.

10. A computer program product comprising code instructions for the execution of a method according to one of claims 1 to 8 for detecting an infectious transmission in a population, when said program is executed on a computer.

11. A storage medium readable by computer item of equipment on which a computer program product comprises code instructions for the execution of a method according to one of claims 1 to 8 for detecting an infectious transmission in a population.
